# EUROPEAN PATENT SPECIFICATION

(11) **EP 2 767 296 B1**
(45) Date of publication and mention of the grant of the patent: **20.07.2016**
(21) Application number: 12839867.4
(22) Date of filing: 11.10.2012
(51) Int. Cl.: A61M 1/14, A61M 1/16

(54) **DIAGNOSTIC METHOD OF DIALYSATE PREPARATION DEVICE, AND DIALYSATE PREPARATION DEVICE**
DIAGNOSEVERFAHREN EINER DIALYSATHERSTELLUNGSVORRICHTUNG UND DIALYSATHERSTELLUNGSVORRICHTUNG
PROCÉDÉ DIAGNOSTIQUE DE DISPOSITIF DE PRÉPARATION DE DIALYSAT, ET DISPOSITIF DE PRÉPARATION DE DIALYSAT

(30) Priority: 11.10.2011 JP 2011224216
(43) Date of publication of application: 20.08.2014
(73) Proprietor: Nipro Corporation, Osaka-shi, Osaka 531-8510 (JP); Shibuya Kogyo Co., Ltd., Kanazawa-shi, Ishikawa 920-8681 (JP)
(72) Inventor: ONO Junichi, Kurashiki-shi Okayama 701-0193 (JP); SAIO Hidetoshi, Osaka-shi Osaka 531-8510 (JP); TAKEUCHI Makoto, Kanazawa-shi Ishikawa 920-8681 (JP)
(74) Representative: Oxley, Rachel Louise
(86) International application number: PCT/JP2012/076275
(87) International publication number: WO 2013/054826

(56) References cited:
- WO-A1-94/11093
- WO-A1-2010/024963
- JP-A- S57 500 565
- JP-A- 2011 092 402
- JP-B2- 3 198 172
- Gert-Inge Bertinsson: "The conductivity of dialysis fluid", EDTNA/ERCA Journal, 1 January 2005 (2005-01-01), XP055187180, Retrieved from the Internet: URL:http://onlinelibrary.wiley.com/store/1 0.1111/j.1755-6686.2005.tb00387.x/asset/j. 1755-6686.2005.tb00387.x.pdf?v=1&t=i99o4qg 1&s=1e757e560c6ae23fdb8000dca169516ec3e9fa c1 [retrieved on 2015-05-04]
- "American National Standard ANSI/AAMI RD52:2004 Dialysate for hemodialysis", , 1 January 2004 (2004-01-01), XP055187177, Retrieved from the Internet: URL:http://www.therenalnetwork.org/home/re sources/CfC/AAMI_RD520408.pdf [retrieved on 2015-05-04]

## Description

### Technical Field

The present invention relates to a diagnosis method for a dialysis fluid preparation apparatus and a dialysis fluid preparation apparatus, and specifically to a diagnosis method for a dialysis fluid preparation apparatus and a dialysis fluid preparation apparatus that mix purified water, an A fluid and a B fluid in predetermined proportions respectively to prepare a dialysis fluid and measure the concentration of the prepared dialysis fluid by concentration measurement means.

### Background Art

Conventionally, there is known a dialysis fluid preparation apparatus including a water feed passage that feeds purified water, an A fluid passage that is connected with the water feed passage and feeds an A fluid containing sodium chloride as a principal ingredient, and a B fluid passage that is connected with the water feed passage and feeds a B fluid composed of a sodium bicarbonate aqueous solution, in order to prepare a dialysis fluid to be used in a dialysis apparatus.

Such a dialysis fluid preparation apparatus includes concentration measurement means provided with a detection unit for electric conductivity downstream of the connecting positions of the A fluid passage and the B fluid passage on the water feed passage, in order to judge whether the prepared dialysis fluid is not abnormal.

Further, as the dialysis fluid preparation apparatus, there is known a dialysis fluid preparation apparatus that, before preparing the dialysis fluid, previously checks the behavior of on-off valves and the like provided therein, for safety improvement in dialysis treatment (Patent Literature 1).

### Prior Art Documents

### Patent Literature

Patent Literature 1: Japanese Patent Laid-Open No. 6-142191

JP57-500565-A discloses a proportioning system for compounding in situ a bicarbonate-containing dialysate suitable for use in a dialysis cell.

JP2011-092402-A discloses a method for monitoring dialysate concentration which measures electrical conductivity of a dialysate prepared by mixing two liquids with dilution water and monitoring the concentration by comparing the electrical conductivity with a predetermined reference value.

WO 94/11093 A1 relates to the use of conductivity meters in the preparation of dialysate.

### Summary of Invention

### Problems to be Solved by the Invention

However, the dialysis fluid preparation apparatus in Patent Literature 1 does not diagnose the accuracy of the concentration measurement means, and there is a possibility that a normal concentration measurement of the dialysis fluid is not performed and the concentration of the prepared dialysis fluid becomes unstable, in the case where the accuracy of the concentration measurement means is decreased, for example, by the adhesion of dialysis fluid deposits to electrodes constituting the detection unit.

In view of such a problem, the present invention provides a diagnosis method for a dialysis fluid preparation apparatus and a dialysis fluid preparation apparatus that do not require special reagents, devices or the like for the diagnosis of the concentration measurement means and allow for a simple diagnosis.

### Means for Solving the Problems

That is, a diagnosis method for a dialysis fluid preparation apparatus according to the invention of claim 1 is a diagnosis method for a dialysis fluid preparation apparatus that includes: a water feed passage to feed purified water; an A fluid passage to feed an A fluid containing sodium chloride as a principal ingredient, the A fluid passage being connected with the water feed passage; a B fluid passage to feed a B fluid composed of a sodium bicarbonate aqueous solution, the B fluid passage being connected with the water feed passage; and concentration measurement means provided with a detection unit downstream of connecting positions of the A fluid passage and the B fluid passage on the water feed passage,
the dialysis fluid preparation apparatus mixing the purified water, the A fluid and the B fluid in predetermined proportions respectively to prepare a dialysis fluid, and measuring a concentration of the prepared dialysis fluid by the concentration measurement means,
in which the diagnosis method for the dialysis fluid preparation apparatus, before preparing the dialysis fluid, feeds the B fluid from the B fluid passage to the water feed passage so as to flow only the B fluid to the water feed passage, and
diagnoses accuracy of the concentration measurement means by measuring an electric conductivity of the B fluid by the concentration measurement means and then comparing the measured electric conductivity of the B fluid with a previously set value.

A dialysis fluid preparation apparatus according to the invention of claim 4 is a dialysis fluid preparation apparatus that includes: a water feed passage to flow purified water; a water feed pump provided on the water feed passage; an A fluid passage to feed an A fluid containing sodium chloride as a principal ingredient, the A fluid passage being connected with the water feed passage; an A fluid pump provided on the A fluid passage; a B fluid passage to feed a B fluid composed of a sodium bicarbonate aqueous solution, the B fluid passage being connected with the water feed passage; a B fluid pump provided on the B fluid passage; concentration measurement means provided with a detection unit downstream of connecting positions of the A fluid passage and the B fluid passage on the water feed passage; and control means to control actuations of the pumps, the dialysis fluid preparation apparatus mixing the purified water, the A fluid and the B fluid in predetermined proportions to prepare a dialysis fluid,
in which the control means is provided with a diagnosis unit to diagnose accuracy of the concentration measurement means,
the control means actuates the B fluid pump to perform a fluid sending of only the B fluid to the water feed passage, without performing a fluid sending with the water feed pump and the A fluid pump, and
the diagnosis unit diagnoses the accuracy of the concentration measurement means by comparing an electric conductivity of the B fluid measured by the detection unit of the concentration measurement means with a previously set value.

### Advantageous Effects of Invention

The inventions of claim 1 and claim 4 use the B fluid to be used in the preparation of the dialysis fluid, for diagnosing the accuracy of the concentration measurement apparatus, and therefore do not require special reagents, devices or the like, allowing for a simple diagnosis within the dialysis fluid preparation apparatus including the concentration measurement apparatus.

### Brief Description of Drawings

[Figure 1] Figure 1 is a configuration diagram of a dialysis fluid preparation apparatus according to a first embodiment.
[Figure 2] Figure 2 is a configuration diagram of a personal dialysis apparatus according to a second embodiment.

### Mode for Carrying out the Invention

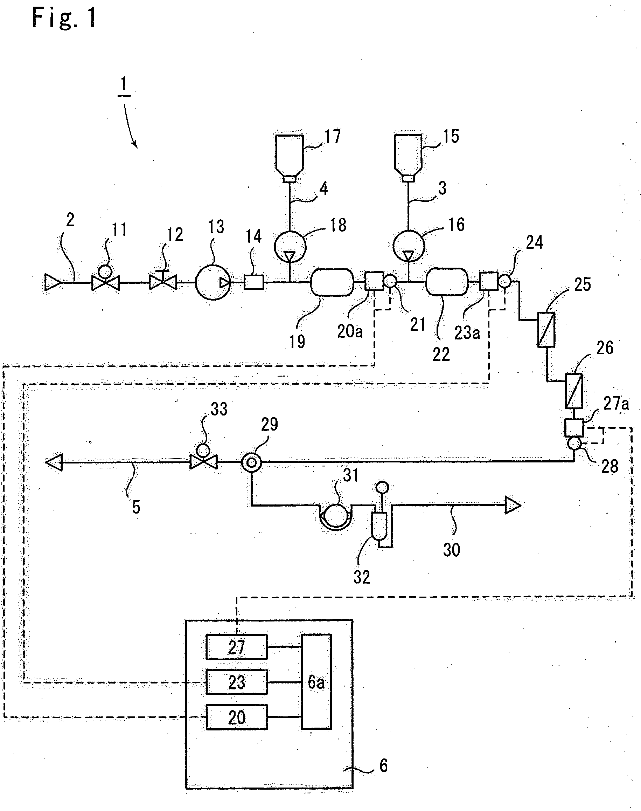

Hereinafter, embodiments shown in the drawings will be described. Figure 1 shows a dialysis fluid preparation apparatus 1 that is installed in hospitals and the like. This dialysis fluid preparation apparatus 1 feeds a prepared dialysis fluid to a plurality of dialysis monitoring apparatuses not shown in the figure, as a so-called dialysis fluid feed apparatus for many persons.

The dialysis fluid preparation apparatus 1 includes a water feed passage 2 that is connected with purified water feed means to feed purified water, which is not shown in the figure, an A fluid passage 3 that is connected with the water feed passage 2 and feeds an A fluid as an undiluted fluid of the dialysis fluid, a B fluid passage 4 that is connected with the water feed passage 2 and feeds a B fluid, and a waste fluid passage 5 that is led from the water.feed passage 2 and drains a poor dialysis fluid. The actuation of solenoid on-off valves and pumps of the dialysis fluid preparation apparatus 1 is controlled by a control means 6.

The A fluid as an undiluted fluid of the dialysis fluid contains sodium chloride as the principal ingredient, and other than sodium chloride, also contains ingredients such as glucose. The ratio is sometimes changed depending on patients using it, and therefore, the concentration of the A fluid itself sometimes varies for each dialysis fluid to be prepared.

On the other hand, the B fluid as an undiluted fluid of the dialysis fluid is composed of an aqueous solution containing only sodium bicarbonate, and the B fluid has a constant concentration regardless of the dialysis fluid to be prepared.

As described in detail later, the dialysis fluid preparation apparatus 1 has a self-check function to previously diagnose the devices constituting the dialysis fluid preparation apparatus 1 before preparing the dialysis fluid, and can switch between a diagnosis mode for performing the self-check and a preparation mode for performing the preparation of the dialysis fluid.

The B fluid passage 4 is connected upstream of the A fluid passage 3 on the water feed passage 2. Upstream of the connecting position of the B fluid passage 4, a first on-off valve 11 to open and close the water feed passage 2, an adjusting valve 12 to adjust the flow rate of the purified water in the water feed passage 2, a purified water pump 13 to send the purified water, and a flow meter 14 to measure the flow rate of the purified water are provided in the order from the upstream side.

The adjusting valve 12, which is a needle valve, adjusts the flow rate of the purified water that the purified water pump 13 sends, by the control of the control means 6. The flow meter 14 measures the flow rate, and feeds back for the flow rate adjustment of the adjusting valve 12.

The A fluid passage 3 has one end connected with an A fluid tank 15 in which the A fluid is pooled, and an A fluid pump 16 to send the A fluid is provided on the A fluid passage 3. Similarly to this, the B fluid passage 4 is connected with a B fluid tank 17 in which the B fluid is pooled, and a B fluid pump 18 to send the B fluid is provided on the B fluid passage 4.

The A fluid pump 16 and the B fluid pump 18 are volume pumps respectively, and include motors not shown in the figure. By rotating these motors at predetermined rotation speeds, it is possible to feed the A fluid and the B fluid to the water feed passage 2 at predetermined flow rates set in the control means 6.

Between the B fluid passage 4 and the A fluid passage 3 on the water feed passage 2, a first buffer tank 19 for temporarily pooling liquid, a detection unit 20a of a first concentration measurement means 20 to measure the concentration of the liquid, and a first temperature measurement means 21 to measure the temperature of the liquid are provided in the order from the upstream side.

Downstream of the A fluid passage 3 on the water feed passage 2, a second buffer tank 22 for temporarily pooling liquid, a detection unit 23a of a second concentration measurement means 23 to measure the concentration of the liquid, a second temperature measurement means 24 to measure the temperature of the liquid, a first filter 25 and second filter 26 to purify the dialysis fluid, a detection unit 27a of a third concentration measurement means 27 to measure the concentration of the liquid, and a third temperature measurement means 28 to measure the temperature of the liquid are provided in the order from the upstream side.

Downstream of the third temperature measurement means 28, a connection port 29 is provided at the boundary with the waste fluid passage 5, and a fluid feed passage 30 is provided at the connection port 29. Further, a fluid sending pump 31 to send the dialysis fluid and a drip chamber 32 are provided on the fluid feed passage 30, and a second on-off valve 33 is provided on the waste fluid passage 5 after the connection port 29.

The first and second buffer tanks 19, 22 form spaces in which the diameter of a part of the water feed passage 2 is enlarged and liquid is stirred. In the first buffer tank 19, the B fluid and the purified water are mixed so that a preparation fluid of the B fluid is prepared. In the second buffer tank 22, the A fluid is mixed with the preparation fluid of the B fluid so that the dialysis fluid is prepared.

The ratio between the B fluid and the purified water in the preparation fluid of the B fluid that is prepared within the first buffer tank 19 is identical to the ratio between the B fluid and the purified water in the dialysis fluid that is prepared by the dialysis fluid preparation apparatus 1. The concentration of the B fluid is previously measured, and is registered in the control means 6.

Also, as for the dialysis fluid that is prepared within the second buffer tank 22, the concentration is previously measured, and is registered in the control means 6. As for this concentration, multiple kinds are registered depending on the A fluid to be used.

The first and second filters 25, 26 remove endotoxins in the prepared dialysis fluid. The endotoxins are surely removed by the two filters.

The first to third concentration measurement means 20, 23, 27 are concentration meters that convert the electric conductivities of the liquids, which are respectively measured by the first to third detection units 20a, 23a, 27a including electrodes, into the concentrations of the liquids, and are incorporated in the control means 6.

The first to third temperature measurement means 21, 24, 28 are disposed adjacent to the first to third detection units 20a, 23a, 27a, respectively, and the first to third concentration measurement means 20, 23, 27 correct the converted concentration values by the measured liquid temperatures.

When the dialysis fluid preparation apparatus 1 is in the preparation mode, the first concentration measurement means 20 measures the electric conductivity of the preparation fluid of the B fluid prepared in the first buffer tank 19, and the second and third concentration measurement means 23, 27 measure the electric conductivity of the dialysis fluid prepared in the second buffer tank 22.

The control means 6 compares these electric conductivities of the preparation fluid of the B fluid and the dialysis fluid that are measured by the first to third concentration measurement means 20, 23, 27, with the previously registered electric conductivities of the preparation fluid of the B fluid and the dialysis fluid, and judges whether these electric conductivities are abnormal.

Then, calcium carbonate is deposited from the preparation fluid of the B fluid and the dialysis fluid, and adheres to the electrodes of the detection units 20a, 23a, 27a so that the measurements become unstable. Therefore, in the embodiment, the dialysis fluid preparation apparatus 1 becomes the diagnosis mode for performing a self-check, before preparing the dialysis fluid.

Thereby, the control means 6 includes a diagnosis unit 6a for performing the self-check, and, in this diagnosis unit 6a, diagnoses the accuracy of the first to third concentration measurement means 20, 23, 27.

Hereinafter, the behavior of the dialysis fluid preparation apparatus 1 having the above configuration will be described. First, in a before-activation state of the dialysis fluid preparation apparatus 1, the A fluid passage 3 and the B fluid passage 4 are connected with the A fluid tank 15 and the B fluid tank 17 in which the A fluid and B fluid to be used in the preparation of the dialysis fluid are pooled, respectively, and the water feed passage 2 is filled with the purified water that has been used as rinse water after washing the water feed passage 2 and in which the A fluid and the B fluid are not mixed.

In this state, once an operator operates an operation panel not shown in the figure to activate the dialysis fluid preparation apparatus 1, it becomes the diagnosis mode to actuate the self-check function and check the behavior of each of the solenoid on-off valves, pumps and measurement means, and therewith, the diagnosis unit 6a of the control means 6 diagnoses the accuracy of the concentration measurement means.

In the following description, a diagnosis method for diagnosing the accuracy of the first to third concentration measurement means 20, 23, 27 will be described.

First, the control means 6 closes the first on-off valve 11 on the water feed passage 2 while opening the second on-off valve 33, stops the purified water pump 13 and the fluid sending pump 31, and actuates the B fluid pump 18 on the B fluid passage 4. At this time, the A fluid pump 16 is being stopped.

Thereby, the B fluid, which is fed from the B fluid tank 17, flows into the water feed passage 2 through the B fluid passage 4, and the purified water filling the water feed passage 2 is pushed to the waste fluid passage 5. Eventually, the water feed passage 2 becomes a state in which the downstream side of the connecting position of the B fluid passage 4 is filled with only the B fluid.

When, after the actuation of the B fluid pump 18, enough time has elapsed for the B fluid to come to at least the detection unit 27a of the third concentration measurement means 27, the control means 6 measures the electric conductivity with the first to third detection units 20a, 23a, 27a of the first to third concentration measurement means 20, 23, 27.

Then, the diagnosis unit 6a compares the measured electric conductivity with a previously set value (for example, 45.4 mS/cm, when a 7% sodium bicarbonate aqueous solution is used as the B undiluted fluid).

If the measured value of the B fluid measured at this time is in a predetermined error range for the previously set value, a diagnosis as being not abnormal is made in the diagnosis unit 6a, and if exceeding the error range, a diagnosis as being abnormal is made.

If each of the concentration measurement means 20, 23, 27 is not abnormal, the control means 6 proceeds with an operation described below, and if being abnormal, the control means 6 gives a warning for the appropriate concentration measurement means.

The control means 6 continuously measures the electric conductivity with the first concentration measurement means 20, after the B fluid pump 18 starts to send the B fluid, and measures the time before the electric conductivity reaches the above predetermined value, that is, the time before the B fluid reaches the first detection unit 20a.

Then, the diagnosis unit 6a compares the measured reaching time of the B fluid with a previously set standard time, and judges whether the B fluid pump 18 is abnormal. If the reaching time is later than the standard time, the fluid sending rate of the B fluid pump 18 is insufficient, and if being earlier, the fluid sending rate is greater than the specification.

After measuring the electric conductivity of the B fluid with the detection units 20a, 23a, 27a of the first to third concentration measurement means 20, 23, 27 in this way, the control means 6 opens the first on-off valve 11 on the water feed passage 2, and further actuates the purified water pump 13 to flow the purified water to the water feed passage 2 at a predetermined flow rate.

On this occasion, the B fluid pump 18 performs the fluid sending at a specified flow rate, and thereby, in the first buffer tank 19 on the water feed passage 2, the B fluid and the purified water are mixed so that the preparation fluid of the B fluid is prepared at a predetermined concentration. This preparation fluid of the B fluid flows to the waste fluid passage 5.

Similarly to the case of sending only the B fluid, the control means 6 measures the electric conductivity with the first to third detection units 20a, 23a, 27a, and the diagnosis unit 6a compares the measured values with a previously set value corresponding to the concentration of the preparation fluid of the B fluid.

By measuring the concentrations of the B fluid and the preparation fluid of the B fluid, that is, the liquids with different electric conductivities, in this way, it is possible to diagnose the first to third concentration measurement means 20, 23, 27 more exactly.

After measuring the.electric conductivity of the preparation fluid of the B fluid with the detection units 20a, 23a, 27a of the first to third concentration measurement means 20, 23, 27 in this way, the control means 6 actuates the A fluid pump 16 on the A fluid passage 3 to feed the A fluid to the water feed passage 2.

On this occasion, also, the B fluid pump 18 is actuated, and thereby, in the second buffer tank 22 positioned downstream of the connecting position of the A fluid passage 3, the A fluid is mixed to the preparation fluid of the B fluid so that the dialysis fluid is prepared. This dialysis fluid flows on the water feed passage 2 to the waste fluid passage 5.

The control means 6 measures the electric conductivity of the dialysis fluid with the second detection unit 23a of the second concentration measurement means 23 and the third detection unit 27a of the third concentration measurement means 27, compares it with a previously set value as the electric conductivity of the dialysis fluid, and judges whether the concentration of the dialysis fluid is good or poor.

Further, by measuring the time after the A fluid pump 16 starts to feed the A fluid and before the second detection unit 23a measures the electric conductivity corresponding to the predetermined concentration of the dialysis fluid, it is possible to diagnose the fluid sending rate of the A fluid pump 16.

Thus, the diagnosis of the accuracy of the first to third concentration measurement means 20, 23, 27 is completed, and when the other devices also are not recognized to be abnormal, the control means 6 finishes the diagnosis of the dialysis fluid preparation apparatus 1. The dialysis fluid preparation apparatus 1 switches from the diagnosis mode to the preparation mode, and subsequently prepares the dialysis fluid.

Thus, in the dialysis fluid preparation apparatus 1 according to the above embodiment, by the diagnosis unit 6a provided in the control means 6, it is possible to automatically diagnose the accuracy of the first to third concentration measurement means 20, 23, 27.

Further, the B fluid to be used in the preparation of the dialysis fluid is utilized for diagnosing the first to third concentration measurement means 20, 23, 27, and therefore, special reagents or devices are not required for the diagnosis, and it is possible to simply perform the diagnosis.

On this occasion, by measuring the reaching time before the B fluid reaches the detection unit of the concentration measurement means, it is possible to diagnose also the fluid sending rate of the B fluid pump 18.

In addition, when diagnosing the first to third concentration measurement means 20, 23, 27, by measuring the B fluid and the preparation fluid of the B fluid, which are liquids with different electric conductivities, it is possible to perform an exact diagnosis.

Although, in the above embodiment, the B fluid passage 4 is connected upstream of the A fluid passage 3 on the water feed passage 2, it can be connected downstream of the A fluid passage 3.

That is, it is possible to be configured such that, in Figure 1, reference numeral 3 denotes the B fluid passage, reference numeral 4 denotes the A fluid passage, reference numerals 15, 16 denote the B fluid tank and the B fluid pump respectively, and reference numerals 17, 18 denote the A fluid tank and the A fluid pump respectively.

In the case of such a configuration, for the diagnosis of the accuracy of the first to third concentration measurement means 20, 23, 27, first, by the same procedure as the above described procedure, the B fluid is sent to the second and third detection units 23a, 27a of the second and third concentration measurement means 23, 27, which are positioned downstream of the B fluid passage (3). Thereby, the diagnosis of the second and third concentration measurement means 23, 27 is performed, and whether the B fluid pump (16) is abnormal is judged.

Subsequently, for the diagnosis of the first concentration measurement means 20 disposed between the B fluid passage (3) and the A fluid passage (4), the first on-off valve 11 on the water feed passage 2 is closed while the second on-off valve 33 is maintained in the open state, and the purified water pump 13 and the fluid sending pump 31 are kept stopped. In that state, the A fluid pump (18) is inversely rotated.

Then, the B fluid filling the downstream side of the connecting position of the B fluid passage 3 on the water feed passage 2 flows backward to the first detection unit 20a of the first concentration measurement means 20. Thereby, it is possible to measure the electric conductivity of the B fluid and diagnose the first concentration measurement means 20.

As another method for the diagnosis of the first concentration measurement means 20, in a state in which the second and third concentration measurement means 23, 27 have been diagnosed, the first on-off valve 11 on the water feed passage 2 is closed, and the purified water pump 13 and the fluid sending pump 31 are kept stopped. In that state, the second on-off valve 33 is closed.

Furthermore, once the B fluid pump (16) is actuated and the A fluid pump (18) is inversely rotated at the same speed as the B fluid pump (16), the B fluid flows backward to the first detection unit 20a of the first concentration measurement means 20. Thereby, it is possible to measure the electric conductivity of the B fluid and diagnose the first concentration measurement means 20.

Here, in the case of providing the A fluid passage (4) upstream of the B fluid passage (3) and inversely rotating the A fluid pump (18) in this way, it is preferable to set a limit on the inverse rotation amount of the A fluid pump (18), in order not to enter the B fluid into the A fluid tank (17).

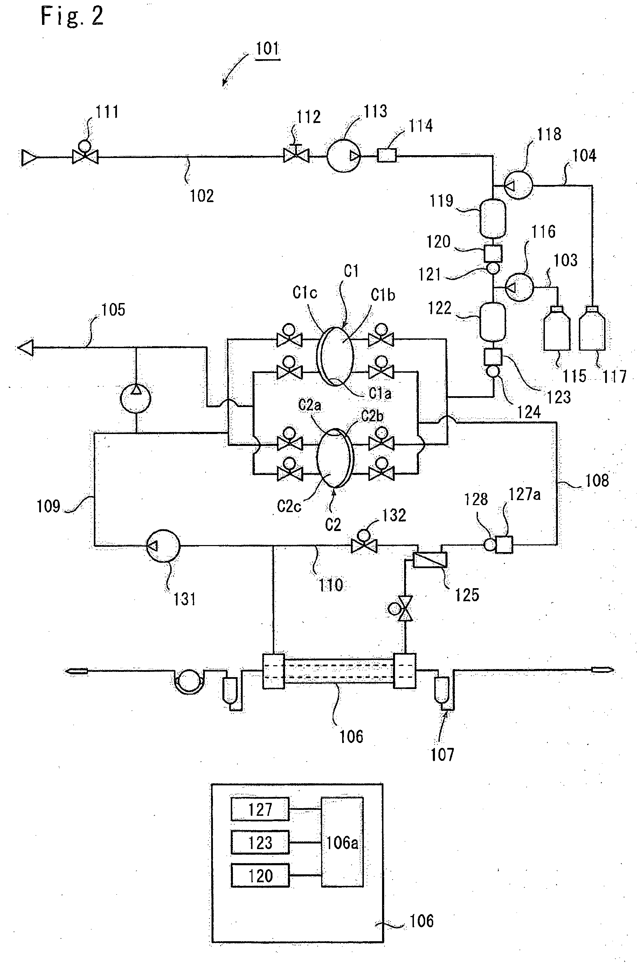

Figure 2, which shows a second embodiment, illustrates a configuration diagram of a so-called personal dialysis apparatus 101. This personal dialysis apparatus 101 has also a function as the diagnosis fluid preparation apparatus 1 shown in Figure 1, and can prepare the diagnosis fluid.

In the following embodiment, as for common constituents with the above first embodiment, reference numerals resulting from adding 100 to the reference numerals used in the first embodiment are used, and detailed descriptions are omitted.

The personal dialysis apparatus 101 includes a dialyzer 106 to perform the dialysis between blood and dialysis fluid, a blood circuit 107 to be connected between the dialyzer 106 and a patient, and first and second chambers C1, C2 to feed fresh dialysis fluid to the dialyzer 106 and retrieve used dialysis fluid.

The first and second chambers C1, C2 are respectively divided into feed chambers C1b, C2b in which the fresh dialysis fluid is pooled by diaphragms C1a, C2a having flexibility, and retrieval chambers C1c, C2c in which the used dialysis fluid is pooled.

The feed chambers C1b, C2b are connected with a water feed passage 102 and a fluid feed passage 108 that feeds the fresh dialysis fluid to the dialyzer 106, and the retrieval chambers C1c, C2c are connected with a waste fluid passage 105 and a retrieval passage 109 that retrieves the used diagnosis fluid from the dialyzer 106. On these passages, various solenoid on-off valves are provided respectively.

The water feed passage 2 in the first embodiment corresponds to the water feed passage 102 and fluid feed passage 108 in the second embodiment, and the detection unit 127a of the third concentration measurement means 127, the third temperature measurement means 128 and the first filter 125 are each provided on the fluid feed passage 108.

The third concentration measurement means 127 and the third temperature measurement means 128 are provided upstream of the first filter 125 on the fluid feed passage 108, and the second filter 26 in the first embodiment is omitted. Further, the fluid sending pump 130 is provided on the retrieval passage 109.

A bypass passage 110 is formed between the first filter 125 and the retrieval passage 109, and by opening a second on-off valve 132 provided on the bypass passage 110, the dialysis fluid judged as a poor concentration is drained.

The personal dialysis apparatus 101 having the above described configuration, itself, is known conventionally, and therefore, detailed descriptions of the behavior are omitted. Similarly to the above first embodiment, the personal dialysis apparatus 101 can diagnose the accuracy of the first to third concentration measurement means 120, 123, 127 with a diagnosis unit 106a provided in a control means 106, and can also diagnose the fluid sending rate of a B fluid pump 118 and an A fluid pump 116.

Here, in the configuration of the second embodiment, also, it is possible to connect the B fluid passage downstream of the A fluid passage, and even if such a configuration, it is possible to diagnose the accuracy of the first to third concentration measurement means 120, 123, 127.

### Reference Signs List

- 1: dialysis fluid preparation apparatus
- 2: water feed passage
- 3: A fluid passage
- 4: B fluid passage
- 5: waste fluid passage
- 6: control means
- 6a: diagnosis unit
- 15: A fluid tank
- 16: A fluid pump
- 17: B fluid tank
- 18: B fluid pump
- 20: first concentration measurement means
- 20a: first detection unit
- 23: second concentration measurement means
- 23a: second detection unit
- 27: third concentration measurement means
- 27a: third detection unit

## Claims

1. A diagnosis method for a dialysis fluid preparation apparatus (1) that comprises: a water feed passage (2) to feed purified water; an A fluid passage (3) to feed an A fluid containing sodium chloride as a principal ingredient, the A fluid passage (3) being connected with the water feed passage (2); a B fluid passage (4) to feed a B fluid composed of a sodium bicarbonate aqueous solution, the B fluid passage (4) being connected with the water feed passage (2); and concentration measurement means (20) provided with a detection unit (20a) downstream of connecting positions of the A fluid passage (3) and the B fluid passage (4) on the water feed passage (2),
the dialysis fluid preparation apparatus (1) mixing the purified water, the A fluid and the B fluid in predetermined proportions respectively to prepare a dialysis fluid, and measuring a concentration of the prepared dialysis fluid by the concentration measurement means (20),
wherein the diagnosis method for the dialysis fluid preparation apparatus (1), before preparing the dialysis fluid, feeds the B fluid from the B fluid passage (4) to the water feed passage (2) so as to flow only the B fluid to the water feed passage (2), and
diagnoses accuracy of the concentration measurement means (20) by measuring an electric conductivity of the B fluid by the concentration measurement means (20) and then comparing the measured electric conductivity of the B fluid with a previously set value.

2. The diagnosis method for the dialysis fluid preparation apparatus (1) according to claim 1,
wherein the diagnosis method for the dialysis fluid preparation apparatus, when measuring the electric conductivity of the B fluid, measures a time after a B fluid pump (18) starts a fluid sending of the B fluid and before the concentration measurement means (20) measures a concentration of the B fluid, the B fluid pump (18) being provided on the B fluid passage (4) and feeding the B fluid to the water feed passage (2), and thereby
diagnoses fluid sending accuracy of the B fluid pump (18).

3. The diagnosis method for the dialysis fluid preparation apparatus (1) according to any one of claims 1 and 2,
wherein the diagnosis method for the dialysis fluid preparation apparatus, after measuring the electric conductivity of the B fluid, mixes the purified water and the B fluid to prepare a preparation fluid of the B fluid by flowing the purified water to the water feed passage (2), and
diagnoses the accuracy of the concentration measurement means (20) by measuring an electric conductivity of the preparation fluid of the B fluid by the concentration measurement means (20) and then comparing the measured electric conductivity of the preparation fluid of the B fluid with a previously set value.

4. A dialysis fluid preparation apparatus (1) that comprises:
a water feed passage (2) to flow purified water;
a water feed pump provided on the water feed passage (2) ;
an A fluid passage (3) to feed an A fluid containing sodium chloride as a principal ingredient, the A fluid passage being connected with the water feed passage (2);
an A fluid pump (16) provided on the A fluid passage (3) ;
a B fluid passage (4) to feed a B fluid composed of a sodium bicarbonate aqueous solution, the B fluid passage (4) being connected with the water feed passage (2);
a B fluid pump (18) provided on the B fluid passage (4);
concentration measurement means (20) provided with a detection unit (20a) downstream of connecting positions of the A fluid passage (3) and the B fluid passage (4) on the water feed passage (2); and
control means (6) to control actuations of the pumps (16, 18), the dialysis fluid preparation apparatus being adapted to mix the purified water, the A fluid and the B fluid in predetermined proportions to prepare a dialysis fluid,
wherein:
the control means (6) is provided with a diagnosis unit (6a) to diagnose accuracy of the concentration measurement means (20);
the control means (6) is adapted to actuate the B fluid pump (18) to perform a fluid sending of only the B fluid to the water feed passage (2), without performing a fluid sending with the water feed pump and the A fluid pump (16); and
the diagnosis unit (6a) is adapted to diagnose the accuracy of the concentration measurement means (20) by comparing an electric conductivity of the B fluid measured by the detection unit (20a) of the concentration measurement means (20) with a previously set value.

## Patentansprüche

1. Diagnoseverfahren für ein Dialyse-Fluid-Herstellungsgerät (1), das Folgendes umfasst: einen Wasserzufuhrdurchlass (2), um gereinigtes Wasser zuzuführen; einen A-Fluid-Durchlass (3), um ein A-Fluid, das Natriumchlorid als einen Hauptbestandteil enthält, zuzuführen, wobei der A-Fluid-Durchlass (3) mit dem Wasserzufuhrdurchlass (2) verbunden ist; einen B-Fluid-Durchlass (4), um ein B-Fluid, das sich aus einer Natriumbikarbonat-wässrigen Lösung zusammensetzt, zuzuführen, wobei der B-Fluid-Durchlass (4) mit dem Wasserzufuhrdurchlass (2) verbunden ist; und ein Konzentrationsmessmittel (20), das mit einer Detektierungseinheit (20a) stromabwärts von Verbindungspositionen des A-Fluid-Durchlasses (3) und des B-Fluid-Durchlasses (4) auf dem Wasserzufuhrdurchlass (2) versehen ist,
wobei das Dialyse-Fluid-Herstellungsgerät (1) das gereinigte Wasser, das A-Fluid und das B-Fluid jeweils in vorbestimmten Verhältnissen mischt, und eine Konzentration des hergestellten Dialyse-Fluids durch das Konzentrationsmessmittel (20) misst,
worin das Diagnoseverfahren für das Dialyse-Fluid-Herstellungsgerät (1) vor dem Herstellen des Dialyse-Fluids das B-Fluid aus dem B-Fluid-Durchlass (4) dem Wasserzufuhrdurchlass (2) zuführt, um lediglich das B-Fluid zum Wasserzufuhrdurchlass (2) strömen zu lassen, und
die Genauigkeit des Konzentrationsmessmittels (20) durch das Messen einer elektrischen Leitfähigkeit des B-Fluids durch das Konzentrationsmessmittel (20) und dann durch das Vergleichen der gemessenen elektrischen Leitfähigkeit des B-Fluids mit einem zuvor festgesetzten Wert diagnostiziert.

2. Diagnoseverfahren für das Dialyse-Fluid-Herstellungsgerät (1) gemäß Anspruch 1,
worin das Diagnoseverfahren für das Dialyse-Fluid-Herstellungsgerät, wenn es die elektrische Leitfähigkeit des B-Fluids misst, eine Zeitspanne nach dem seitens der B-Fluid-Pumpe (18) begonnenen Fluid-Senden des B-Fluids und vor dem Messen einer Konzentration des B-Fluids seitens des Konzentrationsmessmittels (20) misst, wobei die B-Fluid-Pumpe (18) auf dem B-Fluid-Durchlass (4) bereitgestellt ist und das B-Fluid dem Wasserzufuhrdurchlass (2) zuführt, und dadurch
die Fluid-Sende-Genauigkeit der B-Fluid-Pumpe (18) diagnostiziert.

3. Diagnoseverfahren für das Dialyse-Fluid-Herstellungsgerät (1) gemäß einem der Ansprüche 1 und 2;
worin das Diagnoseverfahren für das Dialyse-Fluid-Herstellungsgerät, nach dem Messen der elektrischen Leitfähigkeit des B-Fluids, das gereinigte Wasser und das B-Fluid mischt, um ein Herstellungsfluid des B-Fluids durch das Strömen-Lassen des gereinigten Wassers zum Wasserzufuhrdurchlass (2) herzustellen, und
die Genauigkeit des Konzentrationsmessmittels (20) durch das Messen einer elektrischen Leitfähigkeit des Herstellungsfluids des B-Fluids durch das Konzentrationsmessmittel (20) und dann durch das Vergleichen der gemessenen elektrischen Leitfähigkeit des Herstellungsfluids des B-Fluids mit einem zuvor festgesetzten Wert diagnostiziert.

4. Dialyse-Fluid-Herstellungsgerät (1), das Folgendes umfasst:
einen Wasserzufuhrdurchlass (2), um gereinigtes Wasser hindurchströmen zu lassen;
eine Wasserzufuhrpumpe, die auf dem Wasserzufuhrdurchlass (2) bereitgestellt ist;
einen A-Fluid-Durchlass (3), um ein A-Fluid, das Natriumchlorid als einen Hauptbestandteil enthält, zuzuführen, wobei der A-Fluid-Durchlass mit dem Wasserzufuhrdurchlass (2) verbunden ist;
eine A-Fluid-Pumpe (16), die auf dem A-Fluid-Durchlass (3) bereitgestellt ist;
einen B-Fluid-Durchlass (4), um ein B-Fluid, das sich aus einer Natriumbikarbonat-wässrigen Lösung zusammensetzt, zuzuführen, wobei der B-Fluid-Durchlass (4) mit dem Wasserzufuhrdurchlass (2) verbunden ist;
eine B-Fluid-Pumpe (18), die auf dem B-Fluid-Durchlass (4) bereitgestellt ist; ein Konzentrationsmessmittel (20), das mit einer Detektierungseinheit (20a) stromabwärts von Verbindungspositionen des A-Fluid-Durchlasses (3) und des B-Fluid-Durchlasses (4) auf dem Wasserzufuhrdurchlass (2) versehen ist; und
ein Steuerungsmittel (6) zur Steuerung von Betätigungen der Pumpen (16, 18), wobei das Dialyse-Fluid-Herstellungsgerät angepasst ist, das gereinigte Wasser, das A-Fluid und das B-Fluid in vorbestimmten Verhältnissen zur Herstellung eines Dialyse-Fluids zu mischen,
worin:
das Steuerungsmittel (6) mit einer Diagnoseeinheit (6a) versehen ist, um die Genauigkeit des Konzentrationsmessmittels (20) zu diagnostizieren;
das Steuerungsmittel (6) angepasst ist, die B-Fluid-Pumpe zu betätigen, um ein Fluid-Senden von lediglich dem B-Fluid in den Wasserzufuhrdurchlass (2) auszuführen, ohne ein Fluid-Senden mit der Wasserzufuhrpumpe und der A-Fluid-Pumpe auszuführen; und
die Diagnoseeinheit (6a) angepasst ist, die Genauigkeit des Konzentrationsmessmittels (20) durch ein Vergleichen einer elektrischen Leitfähigkeit des B-Fluids, das durch die Detektierungseinheit (20a) des Konzentrationsmessmittels (20) gemessen wird, mit einem zuvor festgesetzten Wert zu diagnostizieren.

## Revendications

1. Procédé diagnostique pour un appareil (1) de préparation d'un fluide de dialyse qui comprend : un passage d'approvisionnement en eau (2) pour approvisionner de l'eau purifiée, un passage pour un fluide A (3) pour approvisionner un fluide A contenant du chlorure de sodium comme ingrédient principal, le passage pour le fluide A (3) étant relié au passage d'approvisionnement en eau (2), un passage pour un fluide B (4) pour approvisionner un fluide B composé d'une solution aqueuse de bicarbonate de sodium, le passage pour le fluide B (4) étant relié au passage d'approvisionnement en eau (2), des moyens pour mesurer une concentration (20) pourvus d'une unité de détection (20a) en aval de positions de connexion du passage pour le fluide A (3) et du passage pour le fluide B (4) au passage d'approvisionnement en eau (2),
l'appareil (1) de préparation d'un fluide de dialyse mélangeant l'eau purifiée, le fluide A et le fluide B dans des proportions respectivement prédéterminées pour préparer un fluide de dialyse, et mesurant une concentration du fluide de dialyse préparé avec les moyens pour mesurer une concentration (20),
**caractérisé en ce que** le procédé diagnostique pour l'appareil (1) de préparation d'un fluide de dialyse, avant de préparer le fluide de dialyse, approvisionne le fluide B du passage pour le fluide B (4) au passage d'approvisionnement en eau (2) de façon qu'uniquement le fluide B coule au passage d'approvisionnement en eau (2) et
diagnostique la précision des moyens pour mesurer une concentration (20) en mesurant une conductivité du fluide B avec les moyens pour mesurer une concentration (20) et en comparant la conductivité électrique mesurée du fluide B avec une valeur préalablement fixée.

2. Procédé diagnostique pour un appareil (1) de préparation d'un fluide de dialyse selon la revendication 1,
**caractérisé en ce que** le procédé diagnostique pour l'appareil de préparation d'un fluide de dialyse, lorsqu'il mesure la conductivité électrique du fluide B, mesure un temps après qu'une pompe (18) pour le fluide B commence à envoyer le fluide B et avant que les moyens pour mesurer une concentration (20) mesurent une concentration du fluide B, la pompe (18) pour le fluide B étant prévue au passage pour le fluide B (4) et approvisionnant le fluide B au passage pour l'eau (2), et diagnostique par cela la précision d'envoi de fluide de la pompe (18) pour le fluide B.

3. Procédé diagnostique pour un appareil (1) de préparation d'un fluide de dialyse selon l'une quelconque des revendications 1 et 2,
**caractérisé en ce que** le procédé diagnostique pour l'appareil de préparation d'un fluide de dialyse, après avoir mesuré la conductivité électrique du fluide B, mélange l'eau purifiée et le fluide B pour préparer un fluide de préparation du fluide B en laissant coulant l'eau purifiée au passage d'approvisionnement en eau (2) et
diagnostique la précision des moyens pour mesurer une concentration (20) en mesurant une conductivité du fluide B avec les moyens pour mesurer une concentration (20) et en comparant la conductivité électrique mesurée du fluide B avec une valeur préalablement fixée.

4. Appareil (1) de préparation d'un fluide de dialyse qui comprend :
un passage d'approvisionnement en eau (2) pour laisser couler de l'eau purifiée,
une pompe d'approvisionnement en eau prévue au passage d'approvisionnement en eau (2),
un passage pour un fluide A (3) pour approvisionner un fluide A contenant du chlorure de sodium comme ingrédient principal, le passage pour le fluide A (3) étant relié au passage d'approvisionnement en eau (2),
une pompe (16) pour le fluide A prévue au passage pour le fluide A (3),
un passage pour un fluide B (4) pour approvisionner un fluide B composé d'une solution aqueuse de bicarbonate de sodium, le passage pour le fluide B (4) étant relié au passage d'approvisionnement en eau (2),
une pompe (18) pour le fluide B prévue au passage pour le fluide B (4),
des moyens pour mesurer une concentration (20) pourvus d'une unité de détection (20a) en aval de positions de connexion du passage pour le fluide A (3) et du passage pour le fluide B (4) au passage d'approvisionnement en eau (2), et
des moyens d commande (6) pour commander l'actionnement des pompes (16, 18), l'appareil de préparation d'un fluide de dialyse étant adapté pour mélanger l'eau purifiée, le fluide A et le fluide B dans des proportions prédéterminées pour préparer un fluide de dialyse,
**caractérisé en ce que**
les moyens de commande (6) sont pourvus d'une unité diagnostique (6a) pour diagnostiquer la précision des moyens pour mesurer une concentration (20),
les moyens de commande (6) sont adaptés pour actionner la pompe (18) pour le fluide B pour effectuer un envoi de fluide du seul fluide B au passage pour l'eau (2) sans effectuer un envoi de fluide avec la pompe d'approvisionnement en eau et la pompe (16) pour le fluide A, et
l'unité diagnostique (6a) est adaptée pour diagnostiquer la précision des moyens pour mesurer une concentration (20) en comparant une conductivité électrique du fluide B mesurée avec l'unité de détection (20a) des moyens pour mesurer une concentration (20) avec une valeur préalablement fixée.
